# EUROPEAN PATENT APPLICATION

(11) **EP 2 053 060 A1**
(43) Date of publication of application: **29.04.2009**
(21) Application number: 07450189.1
(22) Date of filing: 24.10.2007
(51) Int. Cl.: C07K 14/52, A61P 35/00, A61K 38/19

(54) **SDF-1-based glyocosaminoglycan antagonists and methods of using same**

(71) Applicant: Protaffin Biotechnologie AG, 8020 Graz (AT)
(72) Inventor: Kungl, Andreas, 8045 Graz (AT); Werner, Isa, 8010 Graz (AT); Slingsby, Jason, 1080 Wien (AT); Ali, Simi, Newcastle upon tyne NE20 9DU (GB); Kirby, John A., Newcastle upon Tyne, NE3 1NJ (GB)
(74) Representative: Sonn & Partner Patentanwälte

(57) **Abstract**

The present invention relates to novel mutants of human stromal cell-derived factor-1 which exhibit increased glycosaminoglycan (GAG) binding affinity and inhibited or down-regulated GPCR activity compared to wild type SDF-1, methods for producing these mutants and to their use for preparing medicaments for the treatment of cancer.

SEQUENCE LISTING

<110>  Protaffin Biotechnologie AG

<120>  SDF-1-based glycosaminoglycan antagonists and methods of using
       same

<130>  R 51116

<160>  6

<170>  PatentIn version 3.4

<210>  1
<211>  99
<212>  PRT
<213>  Artificial

<220>
<223>  SDF-1 mutant protein


<220>
<221>  M = methionine or no amino acid residue
<222>  (1)..(1)

<220>
<221>  X = lysine, arginine or no amino acid residue
<222>  (2)..(2)

<220>
<221>  X = proline, glycine or no amino acid residue
<222>  (3)..(3)

<220>
<221>  X = tyrosine or alanine
<222>  (30)..(30)

<220>
<221>  X = serine, arginine, lysine, histidine, asparagine or glutamine
<222>  (40)..(40)

<220>
<221>  X = GRREEKVGKKEKIGKKKRQKKRKAAQKRKN or no amino acid
<222>  (70)..(99)

<400>  1

Met Xaa Xaa Val Ser Leu Ser Tyr Arg Cys Pro Cys Arg Phe Phe Glu
1               5                   10                  15


Ser His Val Ala Arg Ala Asn Val Lys His Leu Lys Ile Xaa Asn Thr
            20                  25                  30


Pro Asn Cys Ala Leu Gln Ile Xaa Ala Arg Leu Lys Asn Asn Asn Arg
        35                  40                  45


Gln Val Cys Ile Asp Pro Lys Leu Lys Trp Ile Gln Glu Tyr Leu Glu
    50                  55                  60


Lys Ala Leu Asn Lys Gly Arg Arg Glu Glu Lys Val Gly Lys Lys Glu
65                  70                  75                  80


Lys Ile Gly Lys Lys Lys Arg Gln Lys Lys Arg Lys Ala Ala Gln Lys
                85                  90                  95

Arg Lys Asn

<210> 2
<211> 68
<212> PRT
<213> Homo Sapiens

<400> 2

Lys Pro Val Ser Leu Ser Tyr Arg Cys Pro Cys Arg Phe Phe Glu Ser
1               5                   10                  15

His Val Ala Arg Ala Asn Val Lys His Leu Lys Ile Leu Asn Thr Pro
            20                  25                  30

Asn Cys Ala Leu Gln Ile Val Ala Arg Leu Lys Asn Asn Asn Arg Gln
        35                  40                  45

Val Cys Ile Asp Pro Lys Leu Lys Trp Ile Gln Glu Tyr Leu Glu Lys
    50                  55                  60

Ala Leu Asn Lys
65

<210> 3
<211> 69
<212> PRT
<213> Artificial

<220>
<223> SDF-1 mutant

<400> 3

Met Arg Gly Val Ser Leu Ser Tyr Arg Cys Pro Cys Arg Phe Phe Glu
1               5                   10                  15

Ser His Val Ala Arg Ala Asn Val Lys His Leu Lys Ile Lys Asn Thr
            20                  25                  30

Pro Asn Cys Ala Leu Gln Ile Val Ala Arg Leu Lys Asn Asn Asn Arg
        35                  40                  45

Gln Val Cys Ile Asp Pro Lys Leu Lys Trp Ile Gln Glu Tyr Leu Glu
    50                  55                  60

Lys Ala Leu Asn Lys
65

<210> 4
<211> 69
<212> PRT
<213> Artificial

<220>
<223> SDF-1 mutant

<400>  4

Met Arg Gly Val Ser Leu Ser Tyr Arg Cys Pro Cys Arg Phe Phe Glu
1               5                   10                  15

Ser His Val Ala Arg Ala Asn Val Lys His Leu Lys Ile Lys Asn Thr
            20                  25                  30

Pro Asn Cys Ala Leu Gln Ile Lys Ala Arg Leu Lys Asn Asn Asn Arg
        35                  40                  45

Gln Val Cys Ile Asp Pro Lys Leu Lys Trp Ile Gln Glu Tyr Leu Glu
    50                  55                  60

Lys Ala Leu Asn Lys
65

<210>  5
<211>  61
<212>  PRT
<213>  Artificial

<220>
<223>  SDF-1 mutant

<400>  5

Met Cys Pro Cys Arg Phe Phe Glu Ser His Val Ala Arg Ala Asn Val
1               5                   10                  15

Lys His Leu Lys Ile Lys Asn Thr Pro Asn Cys Ala Leu Gln Ile Val
            20                  25                  30

Ala Arg Leu Lys Asn Asn Asn Arg Gln Val Cys Ile Asp Pro Lys Leu
        35                  40                  45

Lys Trp Ile Gln Glu Tyr Leu Glu Lys Ala Leu Asn Lys
    50                  55                  60

<210>  6
<211>  61
<212>  PRT
<213>  Artificial

<220>
<223>  SDF-1 mutant

<400>  6

Met Cys Pro Cys Arg Phe Phe Glu Ser His Val Ala Arg Ala Asn Val
1               5                   10                  15

Lys His Leu Lys Ile Lys Asn Thr Pro Asn Cys Ala Leu Gln Ile Lys
            20                  25                  30

Ala Arg Leu Lys Asn Asn Asn Arg Gln Val Cys Ile Asp Pro Lys Leu
        35                  40                  45

Lys Trp Ile Gln Glu Tyr Leu Glu Lys Ala Leu Asn Lys
    50                  55                  60

## Description

The present invention relates to novel mutants of human stromal cell-derived factor-1 (SDF-1α or SDF-1β or SDF-1gamma or any variants thereof) which exhibit (i) increased glycosaminoglycan (GAG) binding affinity and (ii) inhibited or down-regulated GPCR activity compared to wild type SDF-1, and to their use for therapeutic treatment of cancer.

Chemokines are small (8-11 kD) soluble chemoattractant cytokines. They consist of four families, depending on the relative position of their cysteine residues. The α chemokines have one non-conservative amino acid, separating the first two cysteine residues (CXC-motif), whereas the β-chemokines have their first two cysteines adjacent to each other (CC-motif). Fractalkine is the only representative of a chemokine family with three non-conservative amino acids, separating the first two cysteine residues (CX3C-motif) and lymphotactin the only member of a family with a total of only 2 cysteines (C-motif), compared to 4 cysteines in the other three families (Baggiolini et al., Adv. Immunol,. 55, 97 179 (1994)). Stromal cell-derived factor-1α (SDF-1, CXCL12) belongs to the α-chemokines although it is no typical representative of this class. The sequence identity to other human CXC- and CC-chemokines is only 27% and 21%, respectively. Despite this equally distinct relationship to both large chemokine families, mature SDF-1 shows very high similarities between different species. With approx. 99% sequence identity between human and murine and 100% amino acid sequence identity between human and feline SDF-1, it is the most conserved cytokine known so far (Shirozu et al., Genomics, 28, 495-500 (1995)). Orthologues have actually been found in agnatha, the most primitive vertebrate branch. Another characteristic of SDF-1 is its gene SCYB12, which is located on chromosome 10, whereas all other CXC chemokines and most CC chemokines are clustered on chromosome 4 and chromosome 17.

SCYB12 contains four exons. SDF-1α, the most common splice isoform, is derived from exon 1-3, while SDF-1β also contains additional sequence from exon 4 resulting in four additional amino acids at the C-terminus. The three dimensional structure of SDF-1 shows a core of three anti-parallel β-strands and a C-terminal α-helix (Crump et al., EMBO J., 16, 6996-7007 (1997)).

In contrast to the ternary properties, the quaternary structure has always been controversial. A conflict of statements was evident between a monomeric form in NMR-Studies, a dimeric form in crystallographic measurements or a supposed monomer-dimer equilibrium according to sedimentation velocity ultracentrifugation and dynamic light scattering. It was found out later, that SDF-1α in fact exists in a monomer-dimer equilibrium and that dimer association is strongly dependent on both the presence of stabilizing counterions and the solution pH. It was further found that glycosaminoglycans (GAGs) promote SDF-1α dimer formation (Veldkamp et al., Protein Sci., 14, 1071-1081 (2005)).

GAGs are linear polysaccharides comprising repeating disaccharide units that vary in linkage, composition, as well as N- and O-sulfation patterns (Capila & Linhardt, Angew. Chem. Int. Ed. Engl., 41, 391-412 (2002)). Most of them are connected with a protein core thereby forming the so called proteoglycans. Examples of GAGs are heparan sulfate (HS), heparin, keratin sulfate, chondroitin sulfate, dermatan sulfate and hyaluronic acid. Because of the presence of sulfate and carboxylate groups GAGs are highly negatively charged. This drives the binding of the positively charged chemokines which bind soluble glycosaminoglycans as well as GAGs attached on cell surfaces or extracellular matrix. Heparan sulfate, similar to heparin but less charged, is considered fundamental to the biological activity of chemokines. It is produced by most cell types and several core proteins carry HS-chains. For instance syndecan and glypican at the cell surface and agrin and perlecan in the extracellular matrix. It is worth noting that SDF-1α binds to syndecan-4, but not to syndecan-1, syndecan-2, CD44 or beta-glycan. It was shown that Lys24, Lys27 and to a lesser degree Arg41 and Lys43 are essential for the binding of SDF-1 to HS (Sadir et al. J. Biol. Chem., 276, 8288-8296 (2001)). These basic amino acids are located in a small opening between the β-strands of the SDF-1α dimer (Lortat-Jacob et al., Proc. Natl. Acad. Sci., 99, 1229-1234 (2002)).

GAGs lead to the retention of chemokines on cell surfaces and therefore increase the local concentrations of chemokines, in spite of shear forces caused by blood flow. If not for this interaction chemokines would be washed away and could not establish a chemotactic gradient for leukocytes or stem cells to follow. GAG binding is a pre-requisite for in vivo activity. Chemokines with mutations in GAG binding sites are not able to recruit cells in vivo. Structural changes of chemokines leading for example to oligomerization are driven by binding to GAGs. Binding to HS or heparin can protect SDF-1 also from proteolytic cleavage by dipeptidyl peptidase IV (DPP IV)/CD26 in vitro.

The only known receptor for SDF-1 is CXC chemokine receptor 4 (CXCR4) (Y.R. Zou, A.H. Kottmann, M. Kuroda, I. Taniuchi, D.R. Littman, Nature, 393, 595-599 (1998)). Reciprocally SDF-1 is the only known endogenous ligand for CXCR4 (M.K. Schwarz, T.N. Wells, Curr. Opin. Chem. Biol., 3, 407-417 (1999)). CXCR4 belongs to the pertussis toxin sensitive G-protein coupled receptors (GPCR), characterized by seven transmembrane domains. A two-step binding model for SDF-1 and CXCR4 was proposed (M.K. Schwarz, T.N. Wells, Curr. Opin. Chem. Biol., 3, 407-417 (1999)).

Molecular dynamics simulation further confirmed the two-step binding mechanism of SDF-1 to CXCR4. SDF-1 is a highly potent chemokine and attracts lymphocytes and monocytes, as well as hematopoietic stem and progenitor cells (K. Hattori, B. Heissig, K. Tashiro, T. Honio, M. Tateno et al., Blood, 97, 3354-3360 (2001)).

In view of the role of SDF-1 in chemokine attraction and lymphocyte and monocyte attraction and the constant need for new drugs for cancer treatment it is an object of the invention to provide novel proteins for modifying chemokine receptor interaction.

The invention is based on engineering a higher GAG binding affinity into human SDF-1, which can be SDF-1 alpha or SDF-1ß or SDF-1gamma or any variants thereof, and simultaneously knocking out or down the GPCR activity specifically the CXCR4 activity of the chemokine. This is accomplished by introducing basic amino acids into the GAG binding site of SDF-1 in order to increase GAG binding affinity, and by modifying the N-terminus of the chemokine either by truncation of 8 amino acids or by replacing/deleting the first two amino acids. Said SDF-1 mutants exhibit a minimum five-fold improved Kd for standard GAGs (heparin or heparan sulfate) and they show a reduced chemotactic activity in a standard monocytic Boyden chamber. Biophysical and cell biological proof for these characteristics is provided.

Subject matter of the present invention is to inhibit CXCR4-positive cell migration, more specifically stem cells and metastatic cells, by antagonising the GAG interaction with an SDF-1-based mutant in the context of tumour growth and spreading processes. Anti-tumorogenic characteristics are shown in a murine xenotransplant model using human breast cancer cells.

Figures:

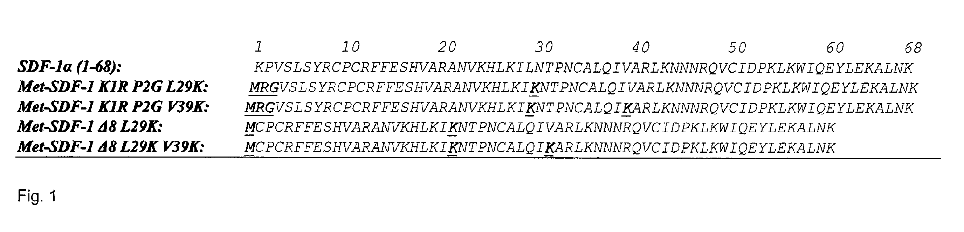

Figure 1: Sequence of SDF-1 mutants, mutations with respect to the wild type chemokine are underlined
Figure 2: Isothermal fluorescence titration of wtSDF-1 and Met-SDF-1Δ8L29KV39K binding to HS.
Figure 3: Monocyte (Thp-1) chemotaxis induced by wtSDF-1 , Met-SDF-1, and Met-SDF-1Δ8L29KV39K.
Figure 4: Inhibtion of human breast cancer cell migration into liver by Met-SDF-1Δ8L29KV39K (designated as "Mutant") as determined in a murine xenotransplant model

It has been shown that increased GAG binding affinity can be introduced by increasing the relative amount of basic amino acids in the GAG binding region (WO 05/054285, incorporated in total herein by reference) leading to a modified protein that acts as competitor with natural GAG binding proteins. This was particularly shown for interleukin-8. The specific location of GAG binding regions and their modification by selectively introducing basic amino acids was not disclosed for SDF-1 (CXCL12) protein.

The present invention now provides a SDF-1 mutant protein with increased GAG binding affinity and reduced GPCR activity compared to wild type SDF-1 protein characterized in that the SDF-1 protein modified in a structure-conserving way by insertion of at least one basic and/or electron donating amino acid or replacement of at least one non-basic amino acid by at least one basic and/or electron donating amino acid and that that at least one amino acid of the first 1 to 10 amino acids of the N-terminal region of the wild type SDF-1 protein is modified by addition, deletion and/or replacement of at least one amino acid.

A protocol for introducing or improving a GAG binding site is for example partially described in WO 05/054285 and can be as follows:
- Identify a region of the protein which is involved in GAG binding
- Design a new GAG binding site by introducing (replacement or insertion) basic or electron donating amino acids, preferably Arg, Lys, His, Asp and Gln residues at any position or by deleting amino acids which interfere with GAG binding
- Check the conformational stability of the resulting mutant protein in silica
- Clone the wild-type protein cDNA (alternatively: purchase the cDNA)
- Use this as template for PCR-assisted mutagenesis to introduce the above mentioned changes into the amino acid sequence
- Subclone the mutant gene into a suitable expression system (prokaryotic or eukaryotic dependent upon biologically required post-translational modifications)
- Expression, purification and characterization of the mutant protein in vitro
   Criterion for an increased GAG binding affinity: K_{d}^{GAG}(mutant) ≤ 10uM.
- check for structural conservation by far-UV CD spectroscopy or 1-D NMR spectroscopy.

A deviation of the modified structure as measured by far-UV CD spectroscopy from wild type structure of less than 30%, preferably less than 20% is defined as structure conserving modification according to the invention.

Electron donating amino acids are those amino acids which donate electrons or hydrogen atoms (Droenstedt definition). Preferably these amino acids can be Asn or Gln. Basic amino acids can be selected from the group consisting of Arg, Lys or His.

According to the present invention the SDF-1 mutant protein can comprise a modification in a structure conserving way wherein the deviation of the modified structure as measured by far-UV CD spectroscopy from wild type SDF-1 structure is less than 30%, preferably less than 20%.

According to an alternative embodiment, the structure conserving modification is located not within the N-terminus of the SDF-1 protein.

The invention covers a SDF-1 mutant protein with increased GAG binding affinity and reduced GPCR activity characterized in that the non-structural region of the SDF-1 protein is modified by insertion or replacement of basic amino acids and N-terminal region of said SDF-1 protein is modified either by truncation of 8 amino acids or by replacing/deleting the first two amino acids.

According to a specific embodiment, the N-terminal amino acids can be selected from the group consisting of Lysine, Arginine, Proline or Glycine.

Further, at least one amino acid at position 29 or 39 can be modified in the SDF-1 mutant protein of the invention. Alternatively, amino acid deletions, substituions or insertions can also be at amino acid positions 24, 25, 26, 27 and/or 41, 42, 43.

Alternatively, the amino acid sequence of the inventive SDF-1 mutant protein can be described by the general formula:
(M)ₙ(X1)ₘ(X2)ₚVSLSYRCPCRFFESHVARANVKHLKI(X3)NTPNCALQI(X4)ARLKNNNR-QVCIDPKLKWIQEYLEKALNK(GRREEKVGKKEKIGKKKRQKK RKAAQKRKN)ₒ
   wherein X1 is a Lysine or Arginine residue,
   wherein X2 is a Proline or Glycine residue,
   wherein X3 is selected of the group consisting of Y and/or A, preferably it is A,
   wherein X4 is selected of the group consisting of S, R, K, H, N and/or Q, preferably it is K,
and wherein n and/or m and/or p and/or o can be either 0 or 1.

According to an alternative embodiment of the invention the SDF-1 mutant protein can contain an N-terminal Met.

A further aspect of the present invention is an isolated polynucleic acid molecule which codes for the inventive protein as described above. The polynucleic acid may be DNA or RNA. Thereby the modifications which lead to the inventive SDF-1 mutant protein are carried out on DNA or RNA level. This inventive isolated polynucleic acid molecule is suitable for diagnostic methods as well as gene therapy and the production of inventive SDF-1 mutant protein on a large scale.

Still preferred, the isolated polynucleic acid molecule hybridises to the above defined inventive polynucleic acid molecule under stringent conditions. Depending on the hybridisation conditions complementary duplexes form between the two DNA or RNA molecules, either by perfectly matching or also comprising mismatched bases (see Sambrook et al., Molecular Cloning: A laboratory manual, 2nd ed., Cold Spring Harbor, N.Y. 1989). Probes greater in length than about 50 nucleotides may accomplish up to 25 to 30% mismatched bases. Smaller probes will accomplish fewer mismatches. The tendency of a target and probe to form duplexes containing mismatched base pairs is controlled by the stringency of the hybridisation conditions which itself is a function of factors, such as the concentrations of salt or formamide in the hybridisation buffer, the temperature of the hybridisation and the post-hybridisation wash conditions. By applying well known principles that occur in the formation of hybrid duplexes conditions having the desired stringency can be achieved by one skilled in the art by selecting from among a variety of hybridisation buffers, temperatures and wash conditions. Thus, conditions can be selected that permit the detection of either perfectly matching or partially matching hybrid duplexes. The melting temperature (Tm) of a duplex is useful for selecting appropriate hybridisation conditions. Stringent hybridisation conditions for polynucleotide molecules over 200 nucleotides in length typically involve hybridising at a temperature 15-25°C below the melting temperature of the expected duplex. For olignucleotide probes over 30 nucleotides which form less stable duplexes than longer probes, stringent hybridisation usually is achieved by hybridising at 5 to 10°C below the Tm. The Tm of a nucleic acid duplex can be calculated using a formula based on the percent G+C contained in the nucleic acids and that takes chain lengths into account, such as the formula
Tm = 81.5-16.6(log[NA⁺])+ 0.41 (% G+C) - (600/N), where N = chain length.

A further aspect relates to a vector comprising an isolated DNA molecule according to the present invention as defined above. The vector comprises all regulatory elements necessary for efficient transfection as well as efficient expression of proteins. Such vectors are well known in the art and any suitable vector can be selected for this purpose.

A further aspect of the present invention relates to a recombinant cell which is transfected with an inventive vector as described above. Transfection of cells and cultivation of recombinant cells can be performed as well known in the art.
Such a recombinant cell as well as any therefrom descendant cell comprises the vector. Thereby a cell line is provided which expresses the SDF-1α mutant protein either continuously or upon activation depending on the vector.

A further aspect of the invention relates to a pharmaceutical composition comprising a SDF-1α mutant protein, a polynucleic acid or a vector according to the present invention as defined above and a pharmaceutically acceptable carrier. Of course, the pharmaceutical composition may further comprise additional substances which are usually present in pharmaceutical compositions, such as salts, buffers, emulgators, colouring agents, etc.

A further aspect of the present invention relates to the use of the SDF-1 protein, a polynucleic acid or a vector according to the present invention as defined above in a method for inhibiting or suppressing the biological activity of the respective wild-type protein. As mentioned above, the SDF-1 mutant protein of the invention will act as an antagonist whereby the side effects which occur with known recombinant proteins will not occur with the inventive SDF-1 mutant protein. In this case this will be particularly the biological activity involved in cancer development.

Therefore, a further use of the SDF-1 protein, a polynucleic acid or a vector according to the present invention as defined above is in a method for producing a medicament for the treatment of cancer. In particular, it will act as antagonist without side effects and will particularly be suitable for the treatment of cancer disease. Therefore, a further aspect of the present invention is also a method for the treatment of cancer diseases, wherein the SDF-1 mutant protein according to the invention, the isolated polynucleic acid molecule or vector according to the present invention or a pharmaceutical preparation according to the invention is administered to a patient.

The present invention is further illustrated by the following examples, however, without being restricted thereto.

### Examples:

### Example 1: Increase in GAG binding affinity

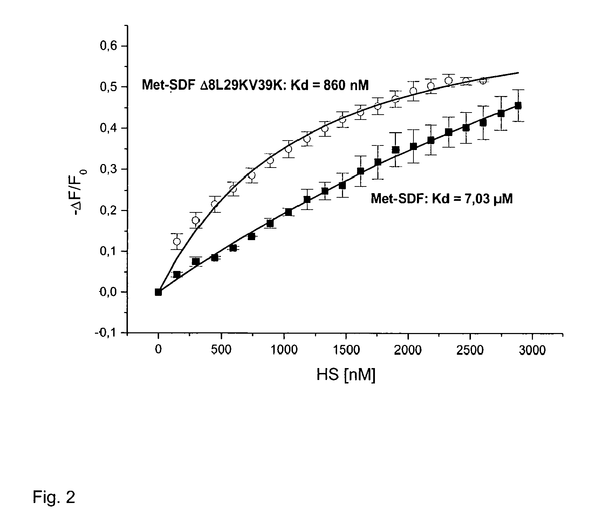

The engineered increased GAG binding affinity of the SDF-1a mutants with respect to wtSDF-1a was determined by isothermal fluorescence titrations (see Figure 2). Steady state fluorescence measurements were performed on a Perkin Elmer (Bacon-sfield, UK) LS50B fluorimeter. The emission of a 180 nM preequilibrated solution of SDF-1a wild type or mutant in PBS (pH 7.2, 125 mM NaCl) upon excitation at 282 nm was recorded over the range of 300-390 nm. Binding isotherms were obtained by coupling the cuvette holder to a thermostat and by the addition of an aliquot of heparan sulfate allowing for an equilibration period of 2 min. The slit width was set at 12 nm for both excitation and emission and the spectra were recorded with 200 nm/min. A 290 nm cut-off was inserted to avoid stray light. The fluorescence spectra were background corrected and the respective areas were integrated between 300 and 390 nm. The normalised mean changes in fluorescence intensity (-ΔF/F0) resulting from three independent experiments were plotted against the ligand concentration. The resulting binding isotherms were fitted by nonlinear regression using the programme Origin (Microcal Inc., Northampton, USA) to the following equation describing a bimolecular association reaction as published previously (Falsone et al. (2001) Biochem Biophys Res Commun 285, 1180-5). A clear improvement in HS affinity by a factor of ca. 10 of the Met-SDF-1 Δ8 L29K V39K mutant with respect to wtSDF-1a was observed.

### Example 2: Knock-down of GPCR activity

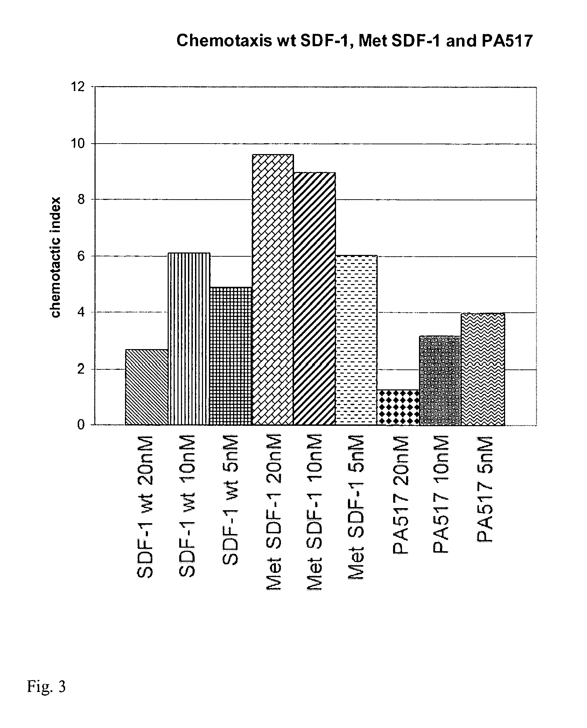

SDF-1a wild type and mutant directed cell migration was investigated using a 48-well Boyden chamber system (Neuroprobe) equipped with 5µm PVP-coated polycarbonate membranes. Wt SDF-a1 and mutant dilutions ranging from 5 nM to 20 nM in RPMI 1640 containing 20 mM HEPES pH 7.3 and 1mg/ml BSA were placed in the lower wells of the chamber in triplicates, including wells with buffer alone. 50 µl of THP-1 cell suspension (European collection of cell cultures) in the same medium at 2 x 10⁶ cells/ml was placed in the upper wells. After a 2 h incubation period at 37°C and 5% CO₂ the upper surface of the filter was washed in Hank's balanced salt solution. The migrated cells were fixed in methanol and stained with Hemacolor® (Merck). The migrated cells of five 400 x magnifications per well were counted and the mean of three independently conducted experiments was background corrected and plotted against the chemokine concentration (see Figure 3; PA517 = Met-SDF-1 Δ8 L29K V39K). The Met-SDF-1 Δ8 L29K V39K mutant exhibited a clearly reduced chemotaxis in vitro.

### Example 3: Inhibition of breast cancer metastasis

For the purpose of investigating the inhibitory activity of SDF-1a mutants on cancer metastasis, a murine xenograft model was used. 10 week old female immunodeficient SCID mice were used for the in vivo experiments. They were kept in isolation care during the whole course of the experiments. The animals were left for a few days after delivery to settle down before the experiments started.

LMD-231 cells were used in this xenograft model. They have been cultured from lung metastasis of mice inoculated with MDA-MB-231. These cells are known to express high levels of CXCR4 and are routinely used in xenograft experiments. These adherent cells were cultured in complete Minimum Essential Medium and complete RPMI mixed in a 50:50 ratio and cell flasks were split in a ratio of 1:3. The cells were cultured at 37°C, 5% CO₂ in a humidified atmosphere.

For in vivo experiment, 120,000 LMD-231 cells were injected into the tail vein of each mouse. Just before injection, the cells were either mixed with PBS for the control group or the mutant chemokine in the desired concentration. (The cells and chemokine were not formally incubated, and the time duration they were mixed together before injection was 5-10 minutes on average.) A total volume of 100µl was injected into each mouse.

On day 28, the animals were killed humanely and organs harvested. Half the liver and a lung from each mouse were snap frozen for future work. The other half of the liver and one lung were fixed in formalin, and later embedded in paraffin wax. Sections were then cut from the liver (and lung). 2 sections from each mouse were stained with a cocktail of cytokeratin markers which are specific for human epithelial cells (breast cancer).

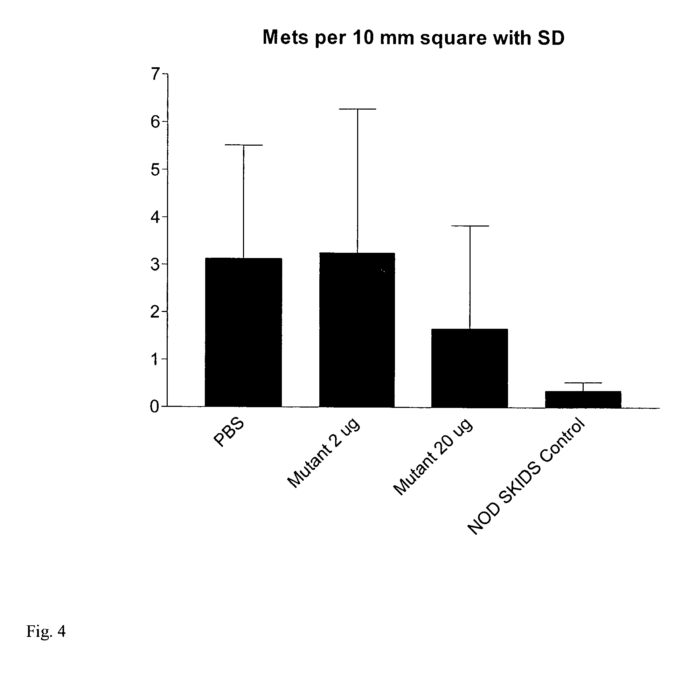

Assessment of liver metastases was accomplished by calculating the total areas of each section using an LCM laser capture microscope and integrated software called. The total numbers of metastases in each section were then counted and average mets per square mm were calculated (Figure 4). The Met-SDF-1 Δ8 L29K V39K mutant showed a clear inhibitory effect on metastasis migration into the liver at 20µg per dose.

## Claims

1. SDF-1 mutant protein with increased GAG binding affinity and reduced GPCR activity compared to wild type SDF-1 protein **characterized in that** the SDF-1 protein is modified in a structure-conserving way by insertion of at least one basic and/or electron donating amino acid or replacement of at least one non-basic amino acid by at least one basic and/or electron donating amino acid and that at least one amino acid of the first 1 to 10 amino acids of the N-terminal region of the wild type SDF-1 protein is modified by addition, deletion and/or replacement of at least one amino acid.

2. SDF-1 mutant protein according to claim 1, **characterized in that** modification in a structure conserving way is a deviation of the modified structure from wild type SDF-1 structure of less than 30%, preferably less than 20% as measured by far-UV-CD spectroscopy.

3. SDF-1 mutant protein according to claims 1 or 2, **characterized in that** the basic amino acids are selected from the group consisting of Arg, Lys, His.

4. SDF-1 mutant protein according to claims 1 or 2, **characterized in that** the electron donating amino acids are selected from the group consisting of Asn or Gln.

5. SDF-1 mutant protein according to any one of claims 1 to 4, **characterized in that** the N-terminal region of said SDF-1 protein is modified by truncation of 8 amino acids.

6. SDF-1 mutant protein according to any one of claims 1 to 5, **characterized in that** the N-terminal region of said SDF-1 protein is modified by replacing and/or deleting the first two amino acids.

7. SDF-1 mutant protein according to any one of claims 1 to 6, **characterized in that** the first two N-terminal amino acids are replaced by amino acids selected from the group consisting of Lysine, Arginine, Proline or Glycine.

8. SDF-1 mutant protein according to any one of claims 1 to 7, **characterized in that** it contains an N-terminal Met.

9. SDF-1 mutant protein according to any one of claims 1 to 8, **characterized in that** at least one amino acid at position 29 or 39 is modified.

10. SDF-1 mutant protein **characterized in that** the amino acid sequence of the modified SDF-1 molecule is described by the general formula:
(M)ₙ(X1)ₘ(X2)ₚVSLSYRCPCRFFESHVARANVKHLKI (X3) NTPNCALQI (X4) ARLKNNNR-QVCIDPKLKWIQEYLEKALNK(GRREEKVGKKEKIGKKKRQKK RKAAQKRKN)ₒ
wherein X1 is a Lysine or Arginine residue,
wherein X2 is a Proline or Glycine residue,
wherein X3 is selected of the group consisting of Y or A, preferably it is A,
wherein X4 is selected of the group consisting of S, R, K, H, N or Q, preferably it is K,
and wherein n and/or m and/or p and/or o can be either 0 or 1.

11. Isolated polynucleic acid molecule, **characterized in that** it codes for a protein according to any one of claims 1 to 10.

12. Isolated polynucleic acid molecule, **characterized in that** it hybridises to the DNA molecule according to claim11 under stringent conditions.

13. Vector, **characterized in that** it comprises an isolated DNA molecule according to claims 11 or 12.

14. Recombinant cell, **characterized in that** it is transfected with a vector according to claim 13.

15. Pharmaceutical composition, **characterized in that** it comprises a protein according to any one of claims 1 to 10 or a polynucleotide according to claims 11 or 12 or a vector according to claim 13 and a pharmaceutically acceptable carrier.

16. Use of SDF-1 mutant protein according to any one of claims 1 to 10 or a polynucleotide according to claims 11 or 12 or a vector according to claim 13 in a method for preparing a medicament for the treatment of cancer.

17. Use according to claim 16, **characterised in that** tumour growth and spreading processes are at least partially inhibited.
